Europäisches Patentamt

**European Patent Office**

Office européen des brevets

(11) Numéro de publication: **0 052 050**
**A2**

## (12) DEMANDE DE BREVET EUROPEEN

(21) Numéro de dépôt: **81401736.4**

(51) Int. Cl.³: **A 45 D 29/00**

(22) Date de dépôt: **29.10.81**

(30) Priorité: **07.11.80 FR 8023844**

(43) Date de publication de la demande: **19.05.82**
**Bulletin 82/20**

(84) Etats contractants désignés: **BE CH DE FR IT LI**

(71) Demandeur: **Plançon, Jacques, 59 rue Jean Jacques Rousseau, F-94290 Villeneuve le Roi (Val-de-Marne) (FR)**
Demandeur: **Plançon, Daniel, 5 Résidence Beauregard, F-91330 Yerres (Essonne) (FR)**
Demandeur: **Plançon, Michel, 16 avenue du Coteau, F-94290 Villeneuve le Roi (Val-de-Marne) (FR)**
Demandeur: **Plançon, Bernard, 59 rue Jean Jacques Rousseau, F-94290 Villeneuve le Roi (Val-de-Marne) (FR)**

(72) Inventeur: **Plançon, Jacques, 59 rue Jean Jacques Rousseau, F-94290 Villeneuve le Roi (Val-de-Marne) (FR)**
Inventeur: **Plançon, Daniel, 5 Résidence Beauregard, F-91330 Yerres (Essonne) (FR)**
Inventeur: **Plançon, Michel, 16 avenue du Coteau, F-94290 Villeneuve le Roi (Val-de-Marne) (FR)**
Inventeur: **Plançon, Bernard, 59 rue Jean Jacques Rousseau, F-94290 Villeneuve le Roi (Val-de-Marne) (FR)**

(74) Mandataire: **Lepeudry-Gautherat, Thérèse et al, CABINET ARMENGAUD JEUNE CASANOVA et LEPEUDRY 23 boulevard de Strasbourg, F-75010 Paris (FR)**

(54) **Outil de podologie en forme de gouge à lame amovible et lame pour un tel outil.**

(57) L'invention concerne les outils de podologie. Elle se rapporte à un outil comportant un manche 14 et des lames amovibles 12a, 12b. Chaque lame se monte par passage d'une saillie latérale 18 du manche dans une ouverture 26, puis par glissement de la lame dans des gorges 20 du manche. Lorsque la lame est en position, elle est verrouillée sur la saillie 18 et peut être déverrouillée par soulèvement de sa partie arrière, avec un doigt d'une main qui tient le manche, alors que l'autre main de l'utilisateur tire la lame vers l'extérieur.

Application à la podologie.

Outil de podologie en forme de gouge à lame amovible et lame pour un tel outil.

La présente invention concerne un outil de podologie en forme de gouge ayant une lame amovible ainsi que des lames destinées à un tel outil.

Les gouges de podologie actuellement utilisées comportent, en une seule pièce, un manche et une lame. La fabrication de ces gouges nécessite un usinage dans un bloc d'acier inoxydable, puis un estampage de la lame afin qu'elle prenne la forme arrondie voulue et une mise en forme du manche afin que celui-ci soit aplati en direction transversale et puisse être tenu fermement à la main. Après l'estampage de la lame, le bord de coupe doit être formé. Ces outils monobloc sont donc relativement coûteux et il n'est pas question de les jeter après usage. Il faut donc les stériliser régulièrement. En outre, étant donné qu'un pédicure dispose de tout un assortiment de gouges, en général au moins quatre mais le plus souvent une dizaine de dimensions différentes, le jeu d'outils nécessaires est relativement important et coûteux.

On n'a pas encore utilisé de tels outils de podologie en deux parties, comprenant un manche et une lame. On a peut-être considéré que, étant donné les efforts importants exercés longitudinalement et latéralement sur l'outil, le montage amovible de la lame n'était pas possible. L'invention concerne un tel outil de podologie dans lequel les lames sont amovibles tout en étant maintenues fermement sur le manche par un dispositif de fixation qui ne laisse aucun jeu à la lame par rapport au manche et qui permet l'application de forces importantes aussi bien longitudinalement que transversalement.

Plus précisément, l'invention concerne un outil de podologie, du type qui comporte une lame formant un bord de coupe à une première extrémité de l'outil, et un manche. Cet outil est tel que le manche et la lame sont séparables et sont destinés à être maintenus en coopération par un dispositif de fixation amovible. Ce dernier comporte un premier organe de guidage et un premier organe de verrouillage, solidaires de la lame, un second organe de guidage et un second organe de verrouillage, solidaires du manche, et un organe de déverrouillage, solidaire de la lame ou du manche.

De façon avantageuse, le manche porte, vers l'extrémité destinée à coopérer avec la lame, une saillie qui délimite deux gorges sensiblement longitudinales et la lame a une ouverture dont les bords délimitent des languettes longitudinales destinées à être glissées dans les gorges du manche. De préférence, l'espacement des bords des languettes qui guident la lame dans les gorges est tel que plus la lame est enfoncée le long du manche et plus le jeu existant entre le fond des gorges et les languettes est faible afin que, lorsque la lame ne peut plus être enfoncée, elle ne présente aucun jeu latéral par rapport au manche.

Les organes de verrouillage de la lame et du manche comportent avantageusement un premier organe de verrouillage, porté par la lame et délimitant le bord de l'ouverture le plus proche du manche et un organe de verrouillage porté par le manche et formé par une face inclinée de la saillie du manche qui est la plus éloignée du bord de coupe de la lame.

Ainsi, dans un mode de réalisation avantageux, le manche porte une saillie délimitant deux gorges latérales et un plan incliné arrière alors que la lame comporte, depuis l'extrémité opposée au bord de coupe, l'organe de déverrouillage, puis une première partie relativement large de son ouverture qui se rétrécit en une partie plus étroite qui assure le blocage sur le manche.

La lame est avantageusement formée dans un feuillard d'acier inoxydable, par découpe, puis cambrage de la lame et formation du bord de coupe à la meule.

Les lames formées dans un feuillard d'acier inoxydable sont ainsi peu coûteuses et peuvent être jetées après utilisation. Elles peuvent ainsi être livrées sous emballage, à l'état stérile.

L'invention concerne aussi un outil tel que décrit, comprenant un manche et tout un jeu de lames ayant diverses dimensions.

D'autres caractéristiques et avantages de l'invention ressortiront mieux de la description qui va suivre, faite en référence au dessin annexé sur lequel :

- la figure 1 est une perspective d'un outil de podologie selon l'invention ;

- la figure 2 est une coupe suivant la ligne A-A de la figure 1 ;

- la figure 3 est une élévation latérale d'une extrémité du manche de l'outil de la figure 1 ;

- la figure 4 est une perspective analogue à la figure 1, représentant le manche et deux lames séparées du manche ; et

- la figure 5 représente une lame pour outil selon l'invention, dans un emballage la maintenant à l'état stérile.

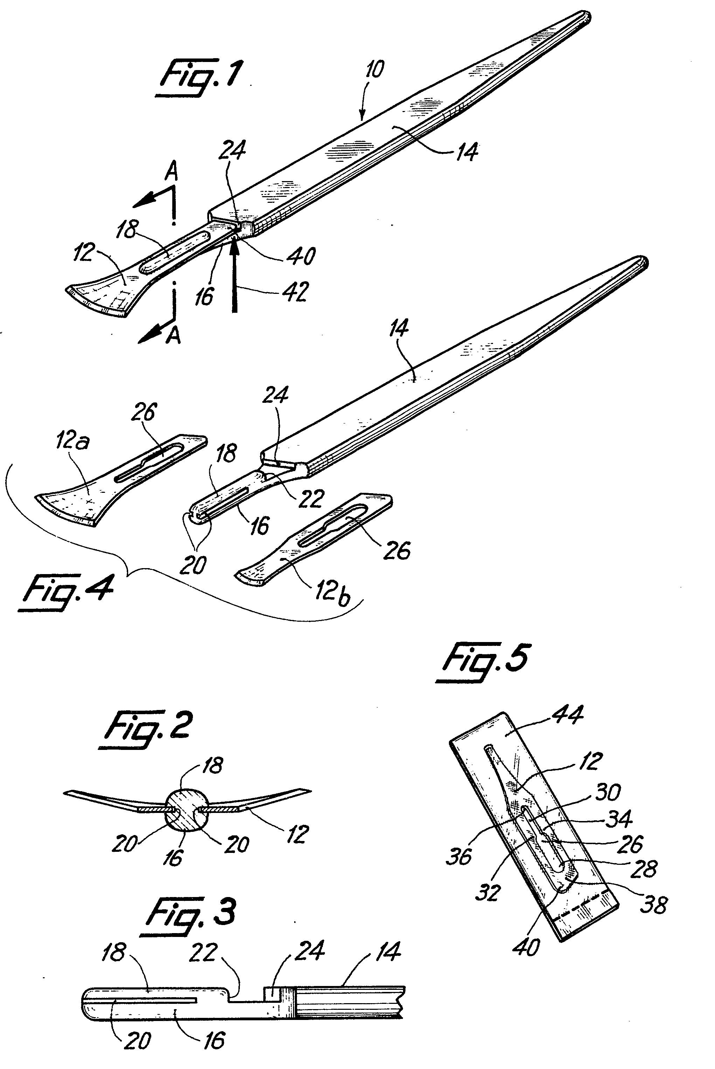

La figure 1 est une perspective d'un outil de podologie selon l'invention. L'outil représenté comporte un manche 10 et une lame 12. Comme indiqué plus clairement sur la figure 4, le manche 10 comporte un corps 14 de forme aplatie, destiné à être saisi par l'utilisateur, et une pointe 16 destinée à retenir la lame. La pointe 16 a une saillie 18 qui dépasse vers le haut sur les figures 1 et 4 de manière qu'un évidement soit délimité entre cette saillie 18 et le corps 14 du manche. Des gorges 20 sont formées le long de la pointe 16. La figure 3 montre clairement que la saillie 18 se termine, du côté du corps 14, par une surface inclinée 22 dont le rôle est indiqué plus en détail dans la suite. En outre, le corps 14 du manche est délimité, du côté de la pointe, par une surface inclinée 24 contre laquelle vient s'appuyer l'extrémité de la lame 12 lorsque celle-ci est soumise à des forces longitudinales excessives.

La lame 12, représentée plus clairement sur la figure 5, a une ouverture 26. Cette dernière a une partie élargie 28, suffisamment grande pour que la saillie 18 de la pointe 16 y passe, et une partie rétrécie 30, délimitée par des languettes 32 et 34. Celles-ci sont destinées à glisser dans les gorges 20 de la pointe 16 du manche. De préférence, les gorges ne sont pas exactement parallèles mais ont des fonds qui se rapprochent légèrement vers l'extrémité externe de la pointe, et les bords des languettes 32 et 34 se rapprochent aussi légèrement du côté du bord de coupe de la lame afin que, lorsque les languettes 32 et 34 sont glissées dans les gorges 20, la lame ait tendance de plus en plus à serrer la pointe 16 du manche. En outre, l'extrémité 36 de l'ouverture peut avoir une largeur encore plus réduite afin qu'elle assure un blocage contre l'extrémité de la pointe 16.

La lame 12 comporte aussi une partie 38 qui ferme l'ouverture 26 du côté du manche. Cette partie 38 a un bord en biais qui correspond à l'inclinaison du bord 24 formé sur le corps 14 du manche 10. En outre, l'extrémité latérale 40 de la lame dépasse latéralement de la pointe 16 afin que, comme indiqué sur la figure 1, elle puisse être soulevée par application d'une force par le bas, comme indiqué par la flèche 42 sur la figure 1.

Lorsqu'une lame 12 est mise en place sur le manche 10, la lame est enfoncée afin que la saillie 18 passe dans la partie élargie 28 de l'ouverture 26. Lorsque les languettes 32 et 34 se trouvent en face des gorges 20, la lame est alors enfoncée longitudinalement par rapport au manche 10 et les languettes 32 et 34 guident la lame dans les gorges 20, par rapport à la pointe 16. A ce moment, la partie 38 d'extrémité de la lame prend appui sur la face externe de la saillie 18 et, comme la lame 12 est élastique, l'introduction et le glissement des languettes 32 et 34 dans les gorges 20 nécessitent l'application d'une certaine force à la lame 12. Lorsque la lame arrive presqu'en position de blocage, la partie 38 qui ferme l'ouverture du côté du manche arrive sur la face inclinée 22 de la saillie et retient ainsi la lame en position sur la pointe 16. L'inclinaison 22 est choisie en fonction des tolérances de fabrication de manière que différentes lames puissent toujours être maintenues par enclenchement derrière la saillie 18.

Lorsqu'une lame doit être retirée, l'utilisateur qui tient le manche d'une main, soulève l'organe 40 de déverrouillage en appliquant avec un doigt, par exemple le pouce, une force indiquée par la référence 42 sur la figure 1. La partie arrière 38 de la lame se dégage alors du plan incliné 22 et, de l'autre main, l'utilisateur tire la lame 12 vers l'extérieur. Celle-ci sort alors sans difficulté, les languettes 32 et 34 glissant dans les gorges 20. L'utilisateur peut alors mettre une autre lame ayant une autre dimension de gouge.

La figure 4 représente ainsi deux lames différentes, une lame 12a ayant un bord de coupe de grande largeur, par exemple de 8 à 15 mm alors que la lame 12b a un bord de coupe de petite dimension, par exemple de 1,5 à 5 mm.

La fabrication de l'outil selon l'invention est très simple. Le manche peut être en toute matière convenable, et il s'agit avantageusement d'une pièce moulée. Il suffit que la résistance mécanique du manche soit suffisante pour le support des lames. Le manche peut donc être formé d'un métal de résistance mécanique suffisante ou même de matière plastique, contenant éventuellement un élément rapporté formant la pointe 16.

Les lames sont avantageusement fabriquées à partir d'un feuillard d'acier inoxydable. Le feuillard est découpé à la forme voulue, essentiellement de manière qu'il délimite l'ouverture 26, le bord arrière et le bord de coupe. Ensuite, les ébauches sont cambrées et il ne reste plus qu'une opération de meulage, formant le bord de coupe, pour que les lames soient terminées. Les lames sont donc peu coûteuses et peuvent n'être utilisées qu'une seule fois. Elles sont alors avantageusement livrées sous emballage individuel, à l'état stérile, comme indiqué par la référence 44 sur la figure 5.

L'outil selon l'invention comprend avantageusement le manche et tout un jeu de lames, par exemple un jeu de 10 lames ayant des bords de coupe dont la largeur varie de 1,5 à 15 mm.

Bien qu'on ait décrit un système particulier d'encliquetage de la lame élastique sur le manche, il est bien entendu que l'invention n'est pas limitée à ce mode de réalisation particulier. Il est cependant essentiel que la lame soit maintenue sans jeu sur le manche, que la lame soit verrouillée en position sur le manche, et qu'elle puisse être facilement déverrouillée.

Il est bien entendu que l'invention n'a été décrite et représentée qu'à titre d'exemple préférentiel et qu'on pourra apporter toute équivalence technique dans ses éléments constitutifs sans pour autant sortir de son cadre.

REVENDICATIONS

1.　　　Outil de podologie, du type qui comporte une lame formant un bord de coupe à une première extrémité de l'outil, et un manche, ledit outil étant caractérisé en ce que

　　　　　- le manche (10) et la lame (12) sont séparables et sont destinés à être maintenus en coopération par un dispositif de fixation amovible, et

　　　　　- le dispositif de fixation amovible comprend

　　　　　　　- un premier organe de guidage (32, 34) et un premier organe de verrouillage solidaires de la lame (12),

　　　　　　　- un second organe de guidage (20) et un second organe de verrouillage (22) solidaires du manche (10), et

　　　　　　　- un organe de déverrouillage (40) solidaire de la lame (12) ou du manche (10).

2.　　　Outil selon la revendication 1, caractérisé en ce que l'organe de guidage du manche (10) comporte des gorges (20), et l'organe de guidage de la lame (12) comporte des languettes (32, 34) destinées à glisser dans les gorges.

3.　　　Outil selon la revendication 2, caractérisé en ce que les languettes (32, 34) de la lame (12) délimitent une ouverture (26) telle que, lorsque la lame est enfoncée sur le manche, le jeu de la lame sur le manche diminue et s'annule lorsque la lame est en position de coopération avec le manche.

4.　　　Outil selon l'une quelconque des revendications précédentes, caractérisé en ce que l'organe de verrouillage du manche (10) est une surface inclinée (22), tournée vers l'extrémité du manche qui est opposée à la lame, et l'organe de verrouillage (38) de la lame (12) est repoussé élastiquement contre la surface inclinée (22) afin qu'il tende à repousser la lame en position de coopération avec le manche.

5.　　　Outil selon l'une quelconque des revendications précédentes, caractérisé en ce que la lame (12) est formée par

    - découpe dans un feuillard élastique, formant une ouverture (26) dans la lame,

    - cambrage du feuillard au moins dans la partie destinée à comporter le bord de coupe, et

    - meulage du bord de coupe.

6.    Outil selon l'une quelconque des revendications précédentes, caractérisé en ce que l'organe de déverrouillage est une partie (40) de la lame qui dépasse latéralement du manche.

7.    Outil selon l'une quelconque des revendications précédentes, caractérisé en ce qu'il comporte un manche (10) et plusieurs lames de configurations différentes (12a, 12b), une seule lame au maximum étant montée sur le manche à un moment donné.

8.    Lame pour outil de podologie selon l'une quelconque des revendications précédentes, caractérisée en ce qu'elle comporte un corps formé de feuillard élastique et délimitant une ouverture (26) ayant une partie élargie (28) et une partie plus étroite (30) disposées dans le prolongement l'une de l'autre, l'ouverture étant interrompue, du côté de sa partie la plus large, par une partie (38) délimitée par un bord extérieur incliné par rapport à la direction longitudinale de la lame, celle-ci comportant en outre un bord de coupe cambré disposé du côté où l'ouverture (26) à sa plus petite dimension.

_Fig.1_

_Fig.4_

_Fig.2_

_Fig.3_

_Fig.5_